# EUROPÄISCHE PATENTSCHRIFT

(11) **EP 3 149 477 B1**
(45) Veröffentlichungstag und Bekanntmachung des Hinweises auf die Patenterteilung: **28.08.2024**
(21) Anmeldenummer: 15725997.9
(22) Anmeldetag: 23.05.2015
(51) Int. Cl.: G01N 33/558, G01N 33/80

(54) **VORRICHTUNG UND VERFAHREN ZUR BESTIMMUNG VON BLUTGRUPPENANTIGENEN MIT EINEM INKOMPLETTEN ANTIKÖRPER**
DEVICE AND METHOD FOR DETECTING BLOOD GROUP ANTIGENS BY MEANS OF AN INCOMPLETE ANTIBODY
DISPOSITIF ET PROCÉDÉ DE DÉTERMINATION D'ANTIGÈNES DE GROUPES SANGUINS AU MOYEN D'UN ANTICORPS INCOMPLET

(30) Priorität: 26.05.2014 DE 102014007851
(43) Veröffentlichungstag der Anmeldung: 05.04.2017
(73) Patentinhaber: Grifols Diagnostic Solutions Inc., Emeryville, CA 94608 (US)
(72) Erfinder: SCHWIND, Peter, 1700 Fribourg (CH); CAESAR, Ariane, 3184 Wünnewil (CH)
(74) Vertreter: ABG Intellectual Property Law, S.L.
(86) Internationale Anmeldenummer: PCT/EP2015/001067
(87) Internationale Veröffentlichungsnummer: WO 2015/180834

(56) Entgegenhaltungen:
- WO-A1-2005/005986
- WO-A1-2005/005986
- WO-A1-2005/005991
- WO-A1-2005/005991
- WO-A1-2008/080544
- WO-A1-2008/080544
- C CUMMEROW ET AL: "Apheresis product identification in the transplant center: development of point-of-care protocols for extended blood typing of stem cell apheresis products", BONE MARROW TRANSPLANTATION, vol. 47, no. 6, 1 June 2012 (2012-06-01), pages 860 - 865, XP055201436, ISSN: 0268-3369, DOI: 10.1038/bmt.2011.182
- SALAMA A ET AL: "Rapid detection of antibodies to immunoglobulin A molecules by using the particle gel immunoassay", VOX SANGUINIS, S. KARGER AG, BASEL, CH, vol. 81, no. 1, 1 July 2001 (2001-07-01), pages 45 - 48, XP002351362, ISSN: 0042-9007, DOI: 10.1046/J.1423-0410.2001.00047.X

## Beschreibung

### Gebiet der Erfindung

Die Erfindung betrifft Vorrichtungen und Verfahren zur Bestimmung von Blutgruppenantigenen mit einem inkompletten Antikörper, insbesondere zur simultanen Bestimmung von Blutgruppenantigenen.

### Stand der Technik

In der blutgruppenserologischen Diagnostik werden allgemein Parameter nachgewiesen, die besonders im Zusammenhang mit Transfusionen bzw. dem Morbus Hämolyticus Neonatorum von Bedeutung sind. Dabei handelt es sich unter anderem um den Nachweis von Antigenen auf der Oberfläche der Erythrozyten, die für die Blutgruppen charakteristisch sind. Weitere wichtige Antigensysteme befinden sich auch auf Thrombozyten, Granulozyten, Lymphozyten, die ebenfalls bei Transfusion und/oder Transplantation eine Rolle spielen.

Bekanntermassen werden zur Bestimmung der Blutgruppenantigene die Erythrozyten der zu testenden Person (Spender oder Empfänger) mit Reagenzien, welche blutgruppenspezifische Antikörper enthalten, zusammengebracht. Üblicherweise handelt es sich um Flüssigkeitstests, bei denen durch Mischen einer Erythrozytenhaltigen Probe mit einer Probe, welche Antikörper enthält, die gegen ein bestimmtes Blutgruppenmerkmal gerichtet sind, ein Testansatz hergestellt wird. Der Testansatz wird dann über einen definierten Zeitraum und unter definierten Bedingungen inkubiert und nach Abschluss der Inkubation oder direkt oder nach einem Zentrifugationsschritt entweder visuell oder mit optischen Methoden auf eine eventuelle Agglutination oder Adsorption der Erythrozyten überprüft. Die vorherrschende Endpunktmessung in der Blutgruppenserologie ist nach wie vor die Hämagglutination. Direkt agglutinierende Antikörper werden in der Blutgruppenserologie auch als komplette Antikörper bezeichnet. Antikörper, welche Erythrozyten nicht direkt agglutinieren können, werden in der Blutgruppenserologie analog als inkomplette Antikörper bezeichnet.

Aus der WO 2005/005991 ist die simultane Bestimmung von Blutgruppenantigenen unter Verwendung eines Lateral-Fluss-Test-Formates bekannt. Den Beispielen der WO 2005/005991 ist die Bestimmung von Blutgruppenantigenen mit IgM-Antikörpern zu entnehmen, die komplette Antikörper darstellen und direkt zur Hämagglutination führen. Die WO-Schrift offenbart jedoch nicht die Bestimmung von Blutgruppenantigenen unter Verwendung eines inkompletten Antikörpers mit Hilfe eines Lateral-Fluss-Tests. Die WO2005/005986 bezieht sich auf eine Vorrichtung und ein Verfahren zur simultanen Durchführung von Blutgruppenbestimmung, Serumgegenprobe und Antikörpersuchtest mit Hilfe eines Lateral- Flow-Assays

Lateral-Fluss-Tests finden heute vielfach Anwendung als Schnelltests, z. B. als Schwangerschaftstests, zur Bestimmung von Infektionsmarkern oder als Drogen-screen. Eine Lateral-Fluss-Test-Anordnung besteht aus einem festen Träger, auf dem eine Aufgabezone für die zu untersuchende Probe aufgebracht ist, eine Trennmembran, auf der Bindungselemente, z. B. Fängerantikörper bzw. -antigene gebunden sind und auf der sich Bindungsreaktionen nachweisen lassen und einem saugfähigen Absorptionsbereich, der die zu untersuchende Probe durch die Trennmembran fliessen lässt.

Testmembranen herkömmlicher Lateral-Fluss-Tests werden in der Regel mit chromatographie-ähnlicher Auftrennung beschrieben. Der Analyt in der Probe bindet spezifisch an die in einer Membran befestigten Bindungselemente, die in der Regel in hintereinanderliegenden bzw. übereinanderstehenden Banden angeordnet als Indikatorzonen vorliegen. Der Bindungskomplex wird durch Indikatorpartikel sichtbar gemacht, welche in der Regel in einem Konjugat-Freisetzungs-Pad eingetrocknet in der Anordnung bereits vorliegen. Das Konjugat-Freisetzungs-Pad ist typischerweise zwischen Aufgabezone und Membran angebracht. Die vorbeschichteten farbigen Indikatorpartikel sind beispielsweise mit einem gegen den gesuchten Analyten gerichteten Antikörper beschichtet.

Die wichtigsten Blutgruppenmerkmale, die heute regelmässig bei prätransfusionellen Untersuchungen am Patienten und bei Spendern abgeklärt werden müssen, sind: A, B, D, C.E.ce.Cw.K.k.Jka.Jkb.Fya.Fyb.M.N.S.s.PI .Lea, Leb, Kpa, Kpb, Lua, Lub. Die zu testenden Antigene bzw. Antigen-Epitope sind beispielhaft solche des ABO-Blutgruppensystems, des Rh-, Kell-, Lewis-Hh-, Duffy-Kidd, MNS-, Lutheran-, P-Systems, der Blutgruppensysteme Diego, Yt, Scianna, Dombrock, Colton,

Chido/Rodgers, Gerbich, Cromer, Knops, Landsteiner-Wiener, Xg, Kx, Indian, Ok, Raph, John Milton Hagen, Langereis, Sid, FORS, JR und/oder LAN, insbesondere A1 , A2, AB, B, D, C, c, E, e, Cw, K, k, M, N, S, s, Jka, Jkb, Fya, Fyb, Kpa, Kpb, Jsa, Jsb, Lea, Leb, Lua, Lub, P1 , 1, H, Xga, U, Vw, Wra, Lan, Vel, Dia und/oder Mia.

Erythrozyten haben aufgrund ihrer negativen Netto-Oberflächenladung und das dadurch ausgeübte Zeta-Potential einen natürlichen statistischen Mindestabstand von ca. 300 Angström zwischen den Zellen. Dieser Mindestabstand kann durch Antikörper der IgM-Klasse aufgrund der Molekülgrösse in physiologischem Milieu überbrückt werden, natürlicherweise jedoch nicht durch Antikörper der IgG-Klasse. Dies bedeutet, dass i.d.R. nur IgM-Klasse Antikörper in der Blutgruppenserologie für eine direkte Endpunkt-Messung durch Hämagglutination zur Verfügung stehen. Direkt agglutinierende Antikörper werden in der Blutgruppenserologie auch als komplette Antikörper bezeichnet (die meisten IgM Antikörper sind komplette Antikörper).

Mit IgG-Antikörpern können nach dem heutigen Stand der Technik Blutgruppen in der Regel nicht durch direkte Hämagglutination nachgewiesen werden. Antikörper, welche Erythrozyten nicht direkt agglutinieren können, werden in der Blutgruppenserologie analog als inkomplette Antikörper bezeichnet (die meisten IgG Antikörper sind inkomplette Antikörper).

Dies führt zu der Situation, dass je nachdem, ob für eine bestimmte Blutgruppeneigenschaft (monoklonale) IgM oder andererseits monoklonale IgG oder polyklonale Antikörper für den Nachweis zur Verfügung stehen, mit sogenannten unterschiedlichen Phasen und Reaktionszeiten und -temperaturen gearbeitet werden muss, was harmonisierte bzw. homologisierte Arbeitsabläufe erschwert.

Sofern IgM Antikörper zur Verfügung stehen, ist häufig eine direkte Bestimmung mit Hämagglutination als Endpunkt ohne Beimengung weiterer Antikörper oder Verstärker oder proteolytischer Enzyme und ohne Inkubation ("immediate spin") möglich. Mit der weit verbreiteten Gel-Technik ist für die Durchführung eines solchen Tests keine Inkubation erforderlich, das Reaktionsgemisch aus zu testenden Erythrozyten und dem Antikörper-Reagenz muss lediglich in die Aufgabezonen der Gelkarte pipettiert werden und 9-10 Minuten in einem neutralen, also nicht Antikörper enthaltenden physiologischen Milieu zentrifugiert werden (z.B. DG Gel Neutral Card von Diagnostic Grifols). In einer anderen Variante derselben Technik sind blutgruppenspezifische Antikörper der IgM-Klasse bereits in die Gel-Matrix eingebracht worden. Dann müssen nur die zu testenden Erythrozyten in die Aufgabezonen der Gelkarte pipettiert werden (z. B. DG Gel ABO RH (2D) von Diagnostic Grifols).

Ist der zur Bestimmung eines bestimmten Blutgruppenmerkmals zur Verfügung stehende Antikörper nicht der IgM-Klasse zugehörig, ist ein Technik-/Phasenwechsel erforderlich, um eine Hämagglutination als Endpunkt möglich zu machen. Dies gilt z.B. für folgende der vorgenannten Merkmale, für die nach dem heutigen Stand der Technik keine kommerziell erhältlichen IgM Antikörper verfügbar sind: k, Fya, Kpa, Kpb, Lua. Folgende weitere Merkmale sind ebenenfalls von Interesse, wie Dia, Jsa, Jsb, Coa, Cob, Wra, Xga. Für den Nachweis keines dieser Antigene sind kommerzielle monoklonale IgM-Antikörper verfügbar.

Da IgG Antikörper in der Regel nicht in der Lage sind, den durch die natürliche Abstoßung vorhandenen Abstand zwischen zwei Erythrozyten zu überwinden, kann durch Reaktion mit einem Antigen-spezifischen IgG Antikörper lediglich eine Sensibilisierung (d. h. Antikörperbindung, aber keine Hämagglutination, ergo kein diagnostischer Endpunkt) der für das jeweilige Antigen positiven Zellen erreicht werden, ohne dass es zum visuell sichtbaren Endpunkt einer Hämagglutination kommt, welche wiederum notwendig für einen einfachen visuellen diagnostischen Nachweis ist: Wenn z. B. ein Erythrozyt, der das Blutgruppenmerkmal Duffy a (Fya) trägt, mit einem Anti-Fya Antikörper der IgG-Klasse inkubiert wird, kommt es zur Antikörper-Antigen Reaktion (Sensibilisierung), die jedoch nicht zum sichtbaren Endpunkt der Hämagglutination führt. Um diesen zu erreichen, müssen die sensibilisierten Zellen zusätzlich mit einem klassenspezifischen Antikörper (im vorliegenden Fall Anti-IgG) inkubiert werden, mit dessen Hilfe die mit IgG Antikörpern sensibilisierten Zellen überbrückt und der Endpunkt der Hämagglutination hergestellt werden kann (Indirekter Coombs Test). Die Gel-Technik, die für diesen Zweck weit verbreitet ist, benötigt für diesen Test eine Inkubations-Zeit von 10-15 Minuten bei 37 °C mit nachfolgender Zentrifugation von 9-10 Minuten in einer Anti-Humanglobulin oder Coombs-Karte (z. B. DG Gel Coombs Card von Diagnostic Grifols).

In der ebenfalls weit verbreiteten Röhrchen-Technik muss im Fall des Immediate Spin nicht inkubiert werden, bevor für ca. 20 s zentrifugiert wird. Für den indirekten Coombs Test wird hier zuerst 15 bis 60 Minuten bei 37°C mit dem blutgruppenspezifischen inkompletten Antikörper inkubiert, danach sind mehrere Waschschritte erforderlich, bevor das Antihumanglobulin-Reagenz zugegeben wird und dann für 20s zentrifugiert wird.

Es besteht daher ein Bedarf an einer Vorrichtung und einem Verfahren zur Bestimmung von zellulär-gebundenen Analyten, inbesondere von Blutgruppenantigenen, für die keine standardisierten IgM-Antikörper, insbesondere keine kommerziell erhältlichen IgM-Antikörper verfügbar sind. Ferner besteht ein Bedarf an einer Vorrichtung und einem Verfahren zur simultanen Bestimmung von mindestens zwei zellulärgebundenenen Analyten, wobei nur für einen der beiden zellulär-gebundenen Analyten ein standardisierter IgM-Antikörper wie ein kommerziell erhälticher Antikörper verfügbar ist, so dass im Stand der Technik die Bestimmung beider Analyten Phasen- bzw. Technik-Wechsel erfordert.

### Zusammenfassung der Erfindung

Der Umfang der Erfindung wird durch die Ansprüche definiert.

Gemäß einem ersten Aspekt der vorliegenden Erfindung wird eine Vorrichtung zur Bestimmung eines Blutgruppenantigens in einer flüssigen Probe bereitgestellt, umfassend eine Trennmatrix mit mindestens einer Indikatorzone, dadurch gekennzeichnet, dass die Indikatorzone einen ersten gegen das ein Blutgruppenantigen gerichteten Antikörper oder ein Fragment davon und ein gegen den ersten Antikörper gerichtetes Bindungselement umfasst, wobei der erste Antikörper ein inkompletter Antikörper ist.

Gemäß einer bevorzugten Ausführungsform umfasst die Vorrichtung eine Membran (2) mit einer Aufgabezone (5) zum Auftragen der flüssigen Probe, mindestens einer Indikatorzone, die mit dem Blutgruppenantigens in Wechselwirkung treten kann und mindestens einem Absorptionsbereich (3), welcher die Flüssigkeit nach Passieren der Indikatorzone aufnimmt, wobei die Indikatorzone zwischen der Aufgabezone (5) und einem Absorptionsbereich (3) liegt, dadurch gekennzeichnet, dass die Indikatorzone einen gegen den Blutgruppenantigen gerichteten Antikörper oder ein Fragment davon und ein gegen den ersten Antikörper gerichtetes Bindungselement umfasst, wobei der erste Antikörper ein inkompletter Antikörper ist.

Gemäß einer weiteren bevorzugten Ausführungsform enthält die Vorrichtung mit Gelmaterial befüllte Röhrchen. Die Geltechnik wird zur Bestimmung der Agglutinationsreaktion von Erythrozyten verwendet. Die Gelsäule wirkt als Filter, der die Migration der agglutinierten Erythrozyten gegenüber nicht-agglutinierten Erythrozyten verlangsamt oder aufhält und dadurch eine Trennung bewirkt.

Erfindungsgemäß enthält die Indikatorzone des Gels einen gegen den Blutgruppenantigen gerichteten Antikörper oder ein Fragment davon und ein gegen den ersten Antikörper gerichtetes Bindungselement umfasst, wobei der erste Antikörper ein inkompletter Antikörper ist.

Gemäß einem zweiten Aspekt der vorliegenden Erfindung wird eine Vorrichtung zur simultanen Bestimmung eines ersten und zweiten Blutgruppenantigens in einer flüssigen Probe bereitgestellt, umfassend eine Membran (2) mit einer Aufgabezone (5) zum Auftragen der flüssigen Probe, mindestens zwei Indikatorzonen, die mit den ein Blutgruppenantigen in Wechselwirkung treten können und mindestens einem Absorptionsbereich (3), welcher die Flüssigkeit nach Passieren der Indikatorzone aufnimmt, wobei die Indikatorzonen zwischen der Aufgabezone (5) und dem mindestens einen Absorptionsbereich (3) liegen, dadurch gekennzeichnet, dass (i) die erste Indikatorzone einen ersten gegen den erste Blutgruppenantigen gerichteten Antikörper oder ein Fragment davon und ein gegen den ersten Antikörper gerichtetes Bindungselement umfasst, wobei der erste Antikörper ein inkompletter Antikörper ist und (ii) die zweite Indikatorzone (a) einen ersten gegen den zweiten Blutgruppenantigen gerichteten Antikörper umfasst, wobei der erste Antikörper ein kompletter Antikörper ist; oder (b) einen ersten gegen den zweiten Blutgruppenantigens gerichteten Antikörper umfasst, wobei der erste Antikörper inkomplett ist und ein gegen diesen Antikörper gerichtetes Bindungselement.

Überraschenderweise haben die Erfinder der vorliegenden Anmeldung festgestellt, dass es durch das gemeinsame Aufbringen eines ersten inkompletten Antikörpers und eines zweiten gegen den ersten Antikörper gerichteten Antikörpers in einer Indikatorzone möglich ist, eine Vorrichtung mit einer Trennmatrix, vorzugsweise in Form der Membran einer Lateralflusstest- Vorrichtung oder als Gelmatrix so auszugestalten, dass die Bestimmung eines Blutgruppenantigens mit einem inkompletten Antikörper als Erstantikörper möglich wird. Hierdurch wird die Bestimmung eines Blutgruppenantigens, für den keine standardisierten wie kommerziell erhältliche Antikörper vom IgM-Typ verfügbar sind, überhaupt erst möglich bei Verwendung einer Trennmatrix wie z.B. einer Lateralflusstest-Vorrichtung. Dies führt zu einer enormen Verkürzung der Zeit für die Bestimmung solcher Analyten, die zuvor regelmäßig nur mit Hilfe des indirekten Coombs-Tests bestimmt werden konnten, der einen zusätzlichen Inkubationsschritt erfordert. Die erfindungsgemäße Durchführung ist für den Fachmann auch überraschend, da dieser angenommen hätte, dass z.B. bei Verwendung von anti-lgG-Molekülen als Zweitantikörper diese durch die in Vollblut in hoher Konzentration vorhandenen nicht Analyt-spezifischen IgG-Moleküle neutralisiert werden.

Es bestand daher keine Möglichkeit im Stand der Technik zwei Blutgruppenantigene simultan (also in einer einzigen Lateral-Fluss Vorrichtung oder in einer einzigen Gelkarte mit mehreren Gelröhrchen zur Bestimmung mehrerer Parameter) zu bestimmen, wobei das erste durch einen IgG-Antikörper und das zweite durch einen IgM-Antikörper bestimmt wird, ohne dass es hierbei zu einem notwendigen Technikbzw. Phasenwechsel kommt. Die vorliegende Erfindung bietet daher den Vorteil der simultanen Bestimmung mit einem einzigen Lateral-Flow-Ansatz, die nur einen einzigen homogenen Verfahrensschritt ohne unterschiedliche Milieus und unterschiedliche Inkubationen erfordert.

Gemäß einem dritten Aspekt der vorliegenden Erfindung wird ein Verfahren zur Herstellung der vorstehenden Vorrichtungen bereitgestellt, umfassend das Aufbringen eines ersten gegen einen Blutgruppenantigen gerichteten Antikörpers oder eines Fragmentes davon und eines zweiten gegen den ersten Antikörper gerichteten Antikörpers oder eines Fragmentes davon, wobei der erste Antikörper ein inkompletter Antikörper ist.

Gemäß einem vierten Aspekt der vorliegenden Erfindung wird ein Verfahren zur Bestimmung mindestens eines Blutgruppenantigens bereitgestellt, umfassend das Aufbringen eines ersten gegen einen Blutgruppenantigen gerichteten Antikörpers oder eines Fragmentes davon und eines gegen den ersten Antikörper gerichteten Bindungselementes in der Indikatorzone, wobei der erste Antikörper ein inkompletter Antikörper ist.

Gemäß einem fünften Aspekt der Erfindung wird die Verwendung der erfindungsgemäßen Vorrichtungen zur Analyse von Blut, insbesondere zur Bestimmung von Blutgruppenantigenen bzw.-antigen-Epitopen bereitgestellt.

### Detaillierte Beschreibung der Erfindung

### Definitionen

Im Zusammenhang mit der vorliegenden Erfindung sollen die folgenden Begriffe wie nachstehend erläutert verstanden werden:
Der Begriff"kompletter Antikörper" bedeutet einen Antikörper, der im Milieu physiologischer Kochsalzlösung zur Aggutination von Erythrozyten führt. Komplette Antikörper umfassen IgM-Antikörper oder Fragmente davon, sofern die Fragmente noch zur Agglutination fähig sind. Die IgM-Antikörper können monoklonal oder polyklonal sein.

Der Begriff"inkompletter Antikörper" bedeutet einen Antikörper, der bei Inkubation mit Erythrozyten nicht zu deren Agglutination führt. Inkomplette Antikörper umfassen IgG-Antikörper, IgA-Antikörper, IgD-Antikörper und IgE-Antikörper einschließlich ihrer Subklassen oder Antikörperfragmente davon, sofern die Fragmente noch zur Bindung eines gegen den vollständigen Antikörper gerichteten zweiten Antikörpers fähig sind. Diese Antikörper können monoklonal oder polyklonal sein. Verfahren zur Herstellung von Antikörpern der verschiedenen Klassen sind dem Fachmann bekannt.

Der Begriff "zellulär-gebundener Analyt" bedeutet jedes natürlicherweise an die Oberfläche einer Zelle, vorzugsweise einer humanen Zelle, insbesondere eines Erythrozyten gebundene Molekül. Hierzu gehören beispielsweise Rezeptoren oder Blutgruppenantigene, wobei Blutgruppenantigene bevorzugt sind.

Der Begriff"Blutgruppenantigen" umfasst solche des ABO-Blutgruppensystems, des Rh-, Kell-, Lewis-Hh-, Duffy-Kidd, MNS-, Lutheran-, P-Systems, der Blutgruppensysteme Diego, Yt, Scianna, Dombrock, Colton, Chido/Rodgers, Gerbich, Cromer, Knops, Landsteiner-Wiener, Xg, Kx, Indian, Ok, Raph, John Milton Hagen, Langereis, Sid, FORS, JR und/oder LAN, insbesondere A1 , A2, AB, B, D, C, c, E, e, Cw, K, k, M, N, S, s, Jka, Jkb, Fya, Fyb, Kpa, Kpb, Jsa, Jsb, Lea, Leb, Lua, Lub, P1 , I, H, Xga, U, Vw, Wra, Lan, Vel, Dia und/oder Mia.

### Herstellung der Lateral-Fluss-Vorrichtung

Ein Verfahren, das grundsätzlich zur Herstellung einer Lateral-Fluss-Vorrichtung geeignet ist, ist in der DE 10330982 A1 und WO 2005/005986 wiedergegeben, das jedoch wie nachstehend angegeben verändert wird.

Das Verfahren zur Herstellung einer erfindungsgemäßen Vorrichtung, umfasst das Aufbringen eines ersten gegen einen Blutgruppenantigen gerichteten Antikörpers oder eines Fragmentes davon und eines gegen den ersten Antikörper gerichteten Bindungselementes in der Indikatorzone, wobei der erste Antikörper ein inkompletter Antikörper ist.

Der erste gegen einen Blutgruppenantigen gerichtete Antikörper und das gegen den ersten Antikörper gerichtete Bindungselement können zusammen oder getrennt voneinander auf die Membran im Bereich der Indikatorzone aufgetragen werden. Sofern die beiden getrennt voneinander aufgetragen werden, ist bevorzugt, dass nach dem Auftragen des ersten Antikörpers ein Trocknungsschritt stattfindet, bevor das Bindungselement aufgetragen wird. Die Konzentration des ersten Antikörpers wird empirisch ermittelt und hängt von der Affinität zum zellulärgebundenen Analyten ab. Die Konzentration des Bindungselementes kann in Abhängigkeit von der Konzentration des ersten Antikörpers und dessen Eigenschaften durch Versuchsreihen optimiert werden.

Der zu bestimmende Analyt ist ein Blutgruppenantigen. Besonders bevorzugt ist der erste Antikörper gegen einen Blutgruppenantigen ausgewählt aus den Blutgruppenantigenen A, B, AB, D, C, E, c, e, Cw, K, k, Jka, Jkb, Fya, Fyb, M, N, S, s, P1 , Kpa, Kpb, Lua, Lub, Lea, Leb, Mia, Dia, Jsa, Jsb, Coa, Cob, Wra und Xga, besonders bevorzugt gegen k, S, Fya, Kpa, Kpb, Lua, Lea, Leb, Mia, Lua, Lub, Dia, Jsa, Jsb, Coa, Cob.Wra und Xga gerichtet. Der erste Antikörper, ist hierbei ein inkompletter Antikörper, vorzugsweise ein IgG- oder IgA-Antikörper, besonders bevorzugt ein IgG-Antikörper. Beispielsweise können folgende Antikörper verwendet werden: Anti-Fya: Klon P3TIM (Merck Millipore, VL); Anti-S: Klon P3S13JS123 (Diagast, Ref.78007); Anti-k:Klon P3A1180L67 (Merck Millipore, FA) und Anti-D: Klon ESD-1 (Alba Bioscience).

Vorzugsweise ist das Bindungselement ausgewählt aus einem gegen den ersten Antikörper gerichteten Antikörper oder einem Fragment davon, und einem Lektin oder einem Fragment davon. Besonders bevorzugt ist der gegen den ersten Antikörper gerichtete Antikörper ein anti-lgG-Antikörper. Anti-lgG-Antikörper sind kommerziell verfügbar, besonders bevorzugt sind der Klon MS-278 (Merck Millipore) und als polyklonaler Antikörper, z.B. Anti-Human Globulin Mono-Type oder Anti-IgG (Medion Grifols Diagnostics). Sofern der erste Antikörper ein IgA-Antikörper ist, ist der zweite Antikörper ein anti-lgA-Antikörper. Anti-lgA-Antikörper sind kommerziell verfügbar. Die Anti-IgG bzw. Anti-lgA-Antikörper können vom IgM- oder IgG-Typ sein, bevorzugt ist ein monoklonaler Anti-IgG der IgM-Klasse. Bevorzugte Lektine sind Protein A und Protein G.

Sofern eine Vorrichtung zur simultanen Bestimmung eines ersten und zweiten Blutgruppenantigens nach dem zweiten Aspekt der vorliegenden Erfindung hergestellt werden soll, umfasst (i) die erste Indikatorzone einen ersten gegen den ersten Blutgruppenantigen gerichteten Antikörper oder ein Fragment davon und ein gegen den ersten Antikörper gerichtetes Bindungselement, wobei der erste Antikörper ein inkompletter Antikörper ist und (ii) die zweite Indikatorzone (a) einen ersten gegen den zweiten Blutgruppenantigen gerichteten Antikörper, wobei der erste Antikörper ein kompletter Antikörper ist; oder (b) einen ersten gegen den zweiten Blutgruppenantigen gerichteten Antikörper umfasst, wobei der erste Antikörper inkomplett ist und ein gegen diesen Antikörper gerichtetes Bindungselement.

Vorzugsweise ist der erste Blutgruppenantigen ausgewählt aus den k, Fya, Kpa, Kpb, Lua, Lub, Mia, Dia, Jsa, Jsb, Coa, Cob, Wra, Xga und S und der zweite Blutgruppenantigen ausgewählt ist aus A, B, AB, C, D, E, c, e, Cw, K, Lea, Leb, Jka, Jkb, Fyb, P1 und s.

Der erste gegen den zweiten Blutgruppenantigen gerichtete Antikörper nach Alternative (a) ist ein kompletter Antikörper, insbesondere ein IgM-Antikörper, der direkt zur Hämagglutination führt. Der erste gegen den zweiten Blutgruppenantigen gerichtete Antikörper nach Alternative (b) ist ein inkompletter Antikörper, vorzugsweise ist der erste Antikörper nach Alternative (b) ein IgG- oder IgA-Antikörper. Das gegen den ersten Antikörper gerichtete Bindungselement nach Alternative (b) ermöglicht die Bestimmung durch Hämagglutination. Das Bindungselement ist vorzugsweise ein IgG- oder IgM-Antikörper. Alternativ kann auch ein Lektin wie Protein A oder Protein G verwendet werden.

Die Membran der erfindungsgemäß verwendeten Vorrichtung ist eine poröse Membran. Bevorzugte Membran-Materialien sind beispielsweise Nitrozellulose (z. B. UniSart von Sartorius, HiFlow von Millipore, Whatman, AE99 bzw. FF85/100 von Whatman Schleicher & Schuell), Polyethylen (Lateral Flo von Porex Corporation) oder Nylon (Novylon von CUNO). Vorzugsweise weist die Membran eine möglichst grosse Porengrösse auf, da eine hohe Porosität der Membran das Eindringen insbesondere von zellulären Komponenten der zu bestimmenden Probe, z. B. von Erythrozyten, in die poröse Struktur begünstigt. Von besonderem Vorteil ist der Einsatz aufnehmender Membranen. Die erfindungsgemäße Vorrichtung ist jedoch nicht auf diese Eigenschaften beschränkt. Bevorzugt werden alle Membranen mit einer hohen kapillaren Flussrate, wobei die kapillare Flussrate die Zeit [s] ist, die eine Farblösung braucht, um 40 mm auf einer gegebenen Membran zurückzulegen. Besonders bevorzugt sind Membranen, deren kapillare Flussrate < 100 ist.

In einer bevorzugten Ausführungsform der Erfindung ist in Fliessrichtung hinter der Aufgabezone der erfindungsgemäßen Vorrichtung auf der porösen Membran ein Dichtelement angeordnet. Zur Anwendung kommen zwei- oder dreidimensionale Dichtelemente, die auf der porösen Membran platziert werden und mit denen eine von der übrigen Fläche der porösen Membran separierte Probenauftragszone geschaffen wird. Das Dichtelement hat erfindungsgemäß primär die Wirkung einer Flüssigkeitsbarriere und erlaubt die gerichtete Verteilung von Probenflüssigkeit und Testreagenzien in die poröse Membran. Weiterhin dichtet das Dichtelement erfindungsgemäß die Probenauftragszone ab zur Verhinderung eines unerwünschten Flüssigkeitsübertritts in die anderen Bereiche der Lateral-Fluss-Vorrichtung.

Bevorzugte Ausführungsformen des Dichtelementes sind die Steg- oder Trog- bzw. Trichter-Form. Die Ausformung des Dichtelementes erfolgt durch Schneideprozesse aus dem zur Herstellung des Dichtelementes verwendeten Material. Im Fall der Trichter- bzw. Trogform erhält das Dichtelement eine innere Öffnung, deren bevorzugte Ausführungsvarianten runde, quadratische oder rechteckige, im Fall der Trichterform sich zur Unterseite (Membrankontaktseite) des Dichtelementes verjüngende Formen sind. Bevorzugte Materialien für das Dichtelement sind

Materialien, die nicht wasseraufnehmend (hydrophob) sind. In einer besonderen Ausführungsform sind die Materialien einseitig mit einem Klebstofffilm, beispielsweise einem drucksensitiven bzw. selbsthaftenden Acrylatklebstoff, beschichtet. Somit kann das Dichtelement direkt auf die Oberfläche der porösen Membran geklebt werden. Alternativ kann das Dichtelement mit dem Lateral-Fluss-Gehäuse verbunden, beispielsweise verklebt sein, wobei in dieser Ausführungsform das Lateral-Fluss-Gehäuse das Dichtelement auf die Oberfläche der porösen Membran drückt und damit die Funktionen des Dichtelementes erzielt werden.

Bevorzugte Materialien für die Ausbildung von zweidimensionalen Dichtelementen sind jede Form von Klebebändern oder Klebefolien (z. B. Tesa 4124 von Beiersdorf AG, ARcare 7815 von Adhesives Research). Bevorzugte Materialien für die Ausbildung von dreidimensionalen Dichtelementen sind flexible, geschlossenporige Elastomermaterialien oder flexible Silikonmaterialien mit unterschiedlichen Materialstärken, vorzugsweise 3-5 mm (z. B. Zellkautschuk EPDM140 von Pitzner, Silikonkautschuk oder Vollkautschuk, Härte 40 deg. oder weniger, von Castan).

In einer weiteren bevorzugten Ausführungsform sind aus einem Stück bestehende multiple Dichtelemente mit beispielsweise 20 Einzelkavitäten (Trogform) auf einer Membran angeordnet.

Durch diese Ausgestaltung ist die erfindungsgemäße Vorrichtung in der Lage, flüssige Proben, die Zellen enthalten, wie beispielsweise Vollblut, aufzunehmen, ohne die Zellen dabei abzufiltern. Weiterhin erlaubt das Dichtelement das Auftragen grosser Probenvolumina auf die poröse Membran (Aufgabezone), ohne dass diese überschwemmt wird. Somit unterstützt das Dichtelement die Nutzung der aufnehmenden Eigenschaften der porösen Membran. Weiter garantiert das Dichtelement einen gerichteten Probenfluss. Die erfindungsgemäße Vorrichtung kann jedoch mit oder ohne Dichtelement gut funktionieren.

Für den Absorptionsbereich (Absorption-Pad) der erfindungsgemäßen Vorrichtung werden mechanisch stabile Materialien bevorzugt, vorzugsweise mit Wasserabsorptionskapazitäten von 20-30 g/100 cm² (z.B. Millipore). Der Kontakt zwischen dem Absorptions-Pad und der Lateral Fluss-Membran der erfindungsgemäßen Vorrichtung wird durch Andruck und Überlappung mit der porösen Membran hergestellt. Die genaue Positionierung des Absorptions-Pads auf der Membran wird durch Verkleben des Absorptions-Pads mit der, die Lateral Fluss-Membran tragenden Trägerschicht (backing sheet), erzielt.

In einer weiteren Ausführungsform sind die Komponenten der erfindungsgemäßen Vorrichtung zum Zwecke der mechanischen Verstärkung auf eine Unterlage bzw. Trägerschicht aufgebracht. Die erfindungsgemäße Vorrichtung kann jedoch mit oder ohne Trägerschicht funktionieren. Bevorzugt werden mechanisch stabile und nicht wasseraufnehmende Materialien, vorzugsweise mit Materialstärken von 100 gm oder mehr, die ein- oder zweiseitig mit einem Klebstofffilm, z. B. einem drucksensitiven bzw. selbsthaftenden Acrylatklebstoff, beschichtet sind (z. B. 0.005" Polyester W/GL-187, G & L). Auf der Trägerschicht werden die poröse Membran und das Absorption-Pad fixiert. Im Fall der doppelseitig klebenden Trägerschicht wird die klebende zweite Seite zur Fixierung des Stapels auf weiteren Flächen, z. B. innerhalb der Lateral-Fluss-Gehäuse, eingesetzt.

In einer weiteren Ausführungsform ist die erfindungsgemäße Vorrichtung, entweder mit oder ohne Trägerschicht, auf der die Komponenten der erfindungsgemäßen Vorrichtung aufgebracht sind, in einem Gehäuse integriert, wodurch die Membran-Komponenten aneinander gedrückt werden und das Gehäuse die Dichtelementfunktion unterstützt. Dabei kann die erfindungsgemäße Vorrichtung jedoch mit oder ohne Gehäuse genauso gut funktionieren.

### Bestimmungsverfahren

Das Verfahren wird durch Auftragen einer flüssigen Probe durchgeführt. Die flüssige Probe besteht vorzugsweise aus Blut oder Blutbestandteilen, besonders bevorzugt aus Vollblut, Erythrozyten-Konzentrat, koaguliertem Blut oder Testflüssigkeit, wie Kontrollblut. Die Probe kann hierbei vor dem Auftragen mit einem Puffer verdünnt werden.

Im folgenden wird die Erfindung durch Figuren und Beispiele näher erläutert, ohne sie einzuschränken.

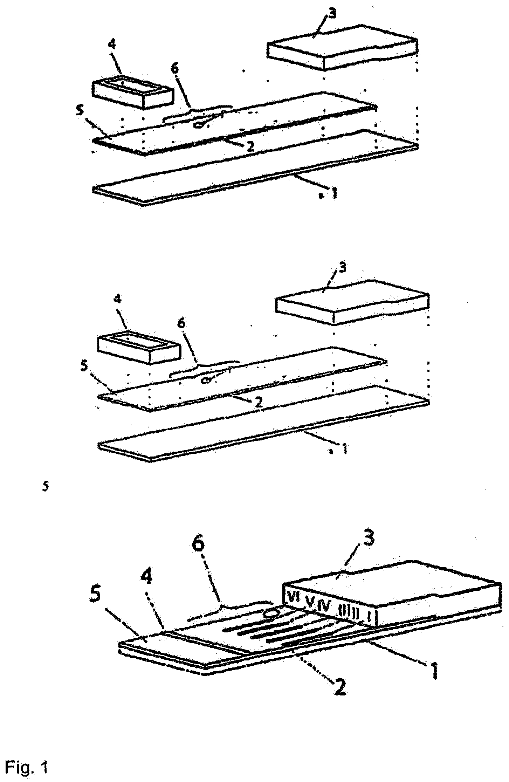

In Fig. 1 wird beispielhaft eine perspektivische Darstellung einer erfindungsgemäßen Vorrichtung für Lateral-Fluss-Tests für die simultane Bestimmung von Blutgruppeantigenen gezeigt. Im vorliegenden Beispiel besteht die Vorrichtung aus einer Trägerschicht 1 , der porösen Membran 2, dem Absorptions-Pad 3 und dem zweidimensionalen, in Stegform ausgeführten oder dreidimensionalen, in Trogform ausgeführten, Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet.

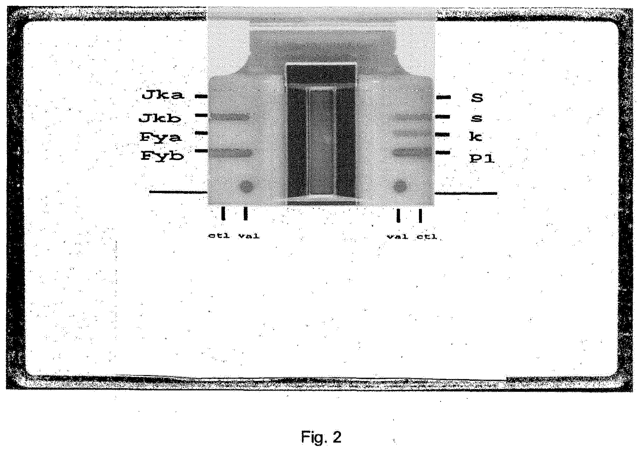

In Fig. 2 wird eine erfolgreiche simultane Bestimmung der Blutgruppenantigene Jka, Jkb, Fya, Fyb, S, s, k und P1 gezeigt. Der Donor ist Jka-Jkb+Fya-Fyb+S-s+k+P1 +. Hierbei wurde die Probe auf die in der Mitte gelegene Aufgabezone aufgetragen. Die Probe durchströmt sowohl die links von der Aufgabezone gelegenen Indikatorzonen als auch die rechts von der Aufgabezone gelegenen Indikatorzonen.

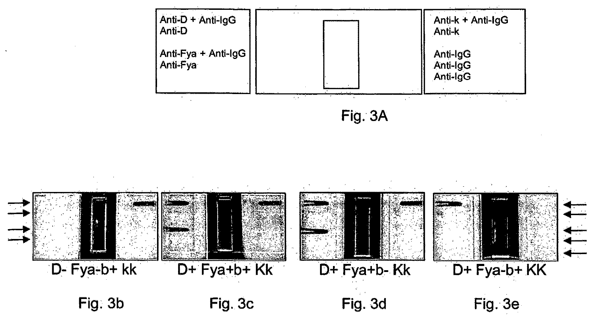

In Fig. 3 ist ein Vergleich einerseits des erfindungsgemäßen Verfahrens mit einem ersten gegen die Blutgruppenantigene D, Fya bzw. k gerichteten IgG-Antikörper und einem hiergegen gerichteten zweiten anti-lgG-Antikörper dargestellt und andererseits eines Vergleichsverfahrens ohne zweiten Antikörper dargestellt. Auf der rechten Seite ist dreimal anti-IgG als weitere Negativ-Kontrolle aufgetragen. Fig. 3a zeigt hierbei das verwendete Dispensierschema. Figuren 3b bis 3e zeigen die
experimentellen Ergebnisse, die mit Proben unterschiedlicher Donoren erhalten wurden.

### Beispiele

### Beispiel 1 : Blutgruppenbestimmung

### Herstellung der Teststreifen :

Die Teststreifen bestehen aus einer in der Mitte der Membran liegenden Aufgabezone, sowie zwei Indikatorzonenbereichen und zwei Absorptionsbereichen in gleichen Abständen zu beiden Seiten der zentralen Aufgabezone. Membranen der Sorte Millipore HiFlow Plus 065 werden in Streifen auf eine Grösse von 19 x 48 mm (Breite/Länge; x/y) für eine 8- bis 10-Bandenausführung zurechtgeschnitten und auf eine Trägerschicht (Backing sheet z.B. von G&L) aufgeklebt. An den zur Aufgabezone distalen Enden der Membran werden zwei mit der Membran um 7mm überlappende 19x17mm grosse Absorptions-Pads (Millipore) aufgeklebt. In zwei linearen Reihen versetzt werden in den Indikatorzonenbereichen 6mm-lange Banden (je 0.6µI) von Lösungen verschiedener blutgruppenspezifischer Antikörper unter Verwendung eines Dispensers, z.B. AD3200 (Biodot), aufgetragen:
Anti-Jka:Klon P3HT7 (Diagast, Ref. 78003); Anti-Jkb: Klon P3 143, (Diagast, Ref. 78004); Anti-Fya: Klon P3TIM + Anti-lgG-Klon MS278 (Merck Millipore, VL+JZ);
Anti-Fyb: Klon SpA264LBg1 (Merck Millipore, FF); Anti-S: Klon P3S13JS123 + Anti-lgG-Klon MS278 (Diagast, Ref.78007+ Merck Millipore, JZ); Anti-s: Klon P3BER (Merck Millipore, FE); Anti-P1 : Klon P3MON2 (Merck Millipore, VN); Anti-k:Klon P3A1180L67 + Anti-lgG-Klon MS278 (Merck Millipore, FA+JZ). Alle Antikörper werden vor der Formulierung ca. 10fach konzentriert.

Die Positionierung des Anti-Jka Antikörpers erfolgt links der Aufgabezone in Position x=3mm/y=9 mm bis y=15. Drei andere Antikörper (Anti-Jkb, Anti-Fya und Anti-Fyb) werden iterierend in Abständen von x=2,5mm parallel zur Position des Anti-Jka Antikörpers dispensiert. Die Positionierung des Anti-S Antikörpers erfolgt rechts der Aufgabezone in Position x=3mm/y=34mm bis y=40. Drei andere Antikörper (Anti-s, Anti-k und Anti-P1 ) werden iterierend in Abständen von x=2,5mm zur Position des Anti-S Antikörpers dispensiert.

Der Anti-Erythrozyten-spezifische Validierungsantikörper (Val = Prozesskontrolle; Rabbit IgG Fraction of Anti-Human RBC, Rockland, 209-4139) wird in x=2,5mm/y=3mm Versetzung zum letzten Bande der Serie der blutgruppenspezifischen Antikörper als Punkt aufgetragen. Der Kontrollpunkt (Ctl = negative Kontrolle; enthält alle Bestandteile der verschiedenen Antikörperformulierungen mit Ausnahme des Antikörpers) wird in y=3mm Versetzung zum Val Punkt aufgetragen. Alle Antikörperlösungen enthalten 1 % BSA und 9,4% APP3 Lösung [32.4% (w/v) D(+) Trehalose dihydrat, 0.055% (v/v) Genapol PF10, 21.8% (v/v) Methanol, PPB Buffer: 15mM Kalium Phosphate Puffer/ 10mM NaCI/ 0.05% (w/v) NaNs]. Die Verdünnung der Antikörper erfolgt in 0.07M Tris/HCI Puffer pH 7, mit Ausnahme von Anti-P1 , welcher in 0.01 M Zitrat Puffer pH 4 verdünnt ist, wie folgt: Anti-Jka 1 :5, Anti-Jkb 1 :5, Anti-Fya 1 :5 + Anti-IgG 1 :25, Anti-S 1 :5 + 1 : 100, Anti-s (small) 1 : 16.7, Anti-k 1 :10 + anti-IgG 1 :100, Anti-P1 1 : 10 und Anti-RBC 1 :10. Die Membranen werden nach dem Dispensieren der Antikörper für 1 Stunde bei 45°C getrocknet und zusammen mit einem Dichtelement in einem Polycarbonatgehäuse (Medion Grifols Diagnostics AG) eingeschweisst.

### Testansatz:

Die Blutproben können in Röhrchen mit gebräuchlichen Antikoagulanzien (z.B. EDTA, CPDA-1 , ACD, Citrat) oder nativ abgenommen werden.

In einem Teströhrchen werden 1 Tropfen (50 µI) antikoaguliertes Vollblut mit 4 Tropfen (200 µI) Diluent F (Medion Grifols Diagnostics) oder 1 Tropfen Erythrozytensediment mit 8 Tropfen (400 µI) Diluent F oder 2 Tropfen (100 µI) der Zellen von koaguliertem Blut mit 2 Tropfen Diluent F gemischt.

Zwei Tropfen (100ul) der resultierenden Suspension werden auf die Aufgabezone der beschriebenen Versuchsanordnung gegeben. Nach 30 Sekunden werden 6 Tropfen (300ul) Diluent F auf die Aufgabezone geben. Nach 5 Minuten werden die Ergebnisse abgelesen und aufgezeichnet.

### Ergebnis:

Der Test ist valid, wenn die anti-RBC Validierungspunkt (val) ein deutlich positives Signal (Roter Punkt) zeigt und der Kontrollpunkt (ctl) ein negatives Ergebnis anzeigt. Die Anwesenheit einer roten Bande zeigt an, dass die getestete Blutprobe positiv für das jeweilige Blutgruppenmerkmal ist. Die Abwesenheit einer Bande in der entsprechenden Position in der Aufgabezone bedeutet, dass die getestete Blutprobe negativ für das entsprechende Blutgruppenmerkmal ist.

Fig. 2 zeigt die erfolgreiche simultane Bestimmung der Blutgruppenantigene Jka, Jkb, Fya, Fyb, S, s, k und P1 gezeigt. Der Donor ist Jka-Jkb+Fya-Fyb+S-s+k+P1 +.

### Beispiel 2: Blutgruppenbestimmung mit dem erfindungsgemäßen Verfahren und Vergleichsbeispiel

Der Teststreifen wurde analog zu Beispiel 1 hergestellt. Als Antikörper wurden verwendet: Anti-D, Klon ESD-1 (Alba), Human IgG; Anti-k (cellano), Klon P3A1180L67 (Millipore), Human IgG; Anti-Fya, Klon P3TIM (Millipore), Human IgG und als Zweitantikörper: Anti-IgG, Klon MS278 (Millipore), Maus-lgM Fig. 3a zeigt das verwendete Dispensierschema. Figuren 3b bis 3e zeigen die experimentellen Ergebnisse, die mit Proben unterschiedlicher Donoren erhalten wurden. Es ist klar erkennbar, dass nur die Bestimmung mit einem ersten gegen das Blutgruppenantigen gerichteten Antikörper der IgG-Klasse und hiergegen gerichteten zweiten Antikörper zu einer deutlich nachweisbaren Bande führt, wohingegen die Bestimmung unter Verwendung des ersten Antikörpers der IgG-Klasse nicht zu einer klar erkennbaren Bande führt. Auf der rechten Seite ist dreimal anti-IgG als weitere Negativkontrolle aufgetragen. Figuren 3b bis 3e zeigen, dass keine Bande bei dieser Negativkontrolle erhalten wurde.

## Patentansprüche

1. Vorrichtung zur Bestimmung eines Blutgruppenantigens in einer flüssigen Probe, umfassend eine Trennmatrix mit mindestens einer Indikatorzone, **dadurch gekennzeichnet, dass** die Indikatorzone einen ersten gegen das ein Blutgruppenantigen gerichteten Antikörper oder ein Fragment davon und ein gegen den ersten Antikörper gerichtetes Bindungselement umfasst, wobei der erste Antikörper ein inkompletter Antikörper ist.

2. Vorrichtung nach Anspruch 1, umfassend eine Membran (2) mit einer Aufgabezone (5) zum Auftragen der flüssigen Probe, mindestens einer Indikatorzone, die mit dem Blutgruppenantigen in Wechselwirkung treten kann und mindestens einem Absorptionsbereich (3), welcher die Flüssigkeit nach Passieren der Indikatorzone aufnimmt, wobei die Indikatorzone zwischen der Aufgabezone (5) und einem Absorptionsbereich (3) liegt, **dadurch gekennzeichnet, dass** die Indikatorzone ein gegen das Blutgruppenantigen gerichteten Antikörper oder ein Fragment davon und ein gegen den ersten Antikörper gerichtetes Bindungselement umfasst, wobei der erste Antikörper ein inkompletter Antikörper ist.

3. Vorrichtung nach Anspruch 1, wobei die Vorrichtung mit Gelmaterial befüllte Röhrchen enthält.

4. Vorrichtung nach einem der Ansprüche 1 bis 3, wobei das Blutgruppenantigen ausgewählt ist aus den Blutgruppenantigenen A, B, AB, D, C, E, c, e, Cw, K, k, Jka, Jkb, Fya, Fyb, M, N, S, s, P1 , Kpa, Kpb, Lua, Lub, Lea, Leb, Mia, Dia, Jsa, Jsb, Coa, Cob, Wra und Xga.

5. Vorrichtung nach Anspruch 4, wobei das Blutgruppenantigen ausgewählt ist aus den Blutgruppenantigenen k, S, Fya, Kpa, Kpb, Mia, Lua, Lub, Dia, Jsa, Jsb, Coa, Cob, Wra und Xga.

6. Vorrichtung nach einem der Ansprüche 1 bis 5, wobei der erste Antikörper ein Antikörper vom IgG- oder IgA-Typ ist, vorzugsweise vom IgG-Typ.

7. Vorrichung nach einem der Ansprüche 1 bis 6, wobei das gegen den ersten Antikörper gerichtete Bindungselement ausgewählt ist aus einem gegen den ersten Antikörper gerichteten Antikörper oder einem Fragment davon, und einem Lektin oder Fragment davon.

8. Vorrichtung nach Anspruch 7, wobei der Antikörper ein anti-IgG oder anti-IgA-Antikörper, vorzugsweise ein monoklonaler anti-IgG-Antikörper der IgM-Klasse, oder das Lektin Protein A oder Protein G ist.

9. Vorrichtung zur simultanen Bestimmung eines ersten und zweiten Blutgruppenantigens in einer flüssigen Probe, umfassend eine Membran (2) mit einer Aufgabezone (5) zum Auftragen der flüssigen Probe, mindestens zwei Indikatorzonen, die mit dem ein Blutgruppenantigen in Wechselwirkung treten können und mindestens einem Absorptionsbereich (3), welcher die Flüssigkeit nach Passieren der Indikatorzone aufnimmt, wobei die Indikatorzonen zwischen der Aufgabezone (5) und dem mindestens einen Absorptionsbereich (3) liegen, **dadurch gekennzeichnet, dass** (i) die erste Indikatorzone einen ersten gegen das erste Blutgruppenantigen gerichteten Antikörper oder ein Fragment davon und ein gegen den ersten Antikörper gerichtetes Bindungselement umfasst, wobei der erste Antikörper ein inkompletter Antikörper ist und
(ii) die zweite Indikatorzone (a) einen ersten gegen das zweite Blutgruppenantigen gerichteten Antikörper umfasst, wobei der erste Antikörper ein kompletter Antikörper ist; oder (b) einen ersten gegen das zweite Blutgruppenantigen gerichteten Antikörper umfasst, wobei der erste Antikörper inkomplett ist und ein gegen diesen Antikörper gerichtetes Bindungselement.

10. Vorrichtung nach Anspruch 9, wobei das erste Blutgruppenantigen ausgewählt ist aus den Blutgruppenantigenen k, Fya, Kpa, Kpb, Lua, Lub, Mia, Dia, Jsa, Jsb, Coa, Cob, Wra, Xga und S und das zweite Blutgruppenantigen ausgewählt ist aus A, B, AB, C, D, E, c, e, Cw, K, Lea, Leb, Jka, Jkb, Fyb, P1 und s.

11. Vorrichtung nach einem der Ansprüche 9 oder 10, wobei der erste Antikörper in der ersten Indikatorzone (i) und/oder der erste Antikörper in der zweiten Indikatorzone (ii) ein Antikörper vom IgG- oder IgA-Typ ist, vorzugsweise vom IgG- Typ.

12. Vorrichung nach einem der Ansprüche 9 bis 11, wobei das gegen den ersten Antikörper gerichtete Bindungselement in der ersten Indikatorzone (i) und/oder in der zweiten Indikatorzone (ii) ausgewählt ist aus einem gegen den ersten Antikörper gerichteten Antikörper oder einem Fragment davon, und einem Lektin oder Fragment davon.

13. Vorrichtung nach Anspruch 12, wobei das Bindungselement ein anti-IgG oder anti-IgA-Antikörper ist oder das Lektin Protein A oder Protein G ist.

14. Vorrichtung nach Anspruch 13, wobei das Bindungselement in der ersten Indikatorzone (i) und/oder der zweite Antikörper in der zweiten Indikatorzone (ii) ein IgM oder IgG-Antikörper ist.

15. Vorrichtung nach einem der Ansprüche 9 bis 14, wobei der erste gegen das erste Blutgruppenantigen gerichtete Antikörper und das gegen den ersten Antikörper gerichtete Bindungselement in der ersten Indikatorzone (i) und/oder der erste gegen das zweite Blutgruppenantigen gerichtete Antikörper und das zweite gegen den ersten Antikkörper gerichtete Bindungselement in der zweiten Indikatorzone (ii) IgG-Antikörper sind.

16. Vorrichtung nach einem der Ansprüche 9 bis 15, wobei die zweite Indikatorzone (ii) einen gegen das zweiten Blutgruppenantigen gerichteten IgM-Antikörper umfasst.

17. Verfahren zur Herstellung einer Vorrichtung nach einem der Ansprüche 1 bis 16, umfassend das Aufbringen eines ersten gegen ein Blutgruppenantigen gerichteten Antikörpers oder eines Fragmentes davon und eines gegen den ersten Antikörper gerichteten Bindungselementes in der Indikatorzone, wobei der erste Antikörper ein inkompletter Antikörper ist.

18. Verfahren zur Herstellung einer Vorrichtung nach Anspruch 17, wobei der erste Antikörper und das Bindungselement getrennt voneinander oder als Gemisch aufgetragen werden.

19. Verfahren zur Bestimmung mindestens eines Blutgruppenantigens, umfassend das Auftragen der Probe auf die Aufgabezone (5) einer Membran (2) der Vorrichtung nach einem der vorangegangenen Ansprüche 1 bis 16, wobei diese Probe in ausreichender Menge vorliegt, um die Probenflüssigkeit dazu zu veranlassen, in Richtung Absorptionsbereich (3) durch die Indikatorzonen zu fliessen und um Blutgruppenantigene in der Probenflüssigkeit dazu zu veranlassen, in den Indikatorzonen einen Komplex zu bilden.

20. Verfahren nach Anspruch 19 , wobei das Verfahren nicht das Inkubieren des Blutgruppenantigens mit einem Antikörper vor dem Auftragen des Blutgruppenantigens auf die Membran umfasst.

21. Verfahren nach Anspruch 19 oder 20, wobei die flüssige Probe aus Blut oder Blutbestandteilen, vorzugsweise aus Vollblut, Erythrozyten-Konzentrat, koaguliertem Blut oder Testflüssigkeit, wie Kontrollblut, besteht.

22. Verwendung der Vorrichtung nach einem der Ansprüche 1 bis 8 oder 9 bis 16 zur Analyse von Blut, insbesondere zur Bestimmung von Blutgruppenantigenen bzw.-antigenEpitopen.

23. Verwendung der Vorrichtung nach Anspruch 22, wobei die Blutgruppenantigene oder Antigenepitope ausgewählt sind aus A, B, AB, D, C, E, c, e, Cw, K, k, Jka, Jkb, Fya, Fyb, M, N, S, s, P1 , Kpa, Kpb, Lua, Lub, Lea, Leb, Mia, Dia, Jsa, Jsb, Coa, Cob, Wra und/oder Xga-Blutgruppenantigene bzw.-antigen-Epitope.

## Claims

1. A device for determining a blood group antigen in a liquid sample, comprising a separation matrix having at least one indicator zone, **characterised in that** the indicator zone comprises a first antibody directed against the blood group antigen, or a fragment thereof, and a binding element directed against the first antibody, wherein the first antibody is an incomplete antibody.

2. The device according to claim 1, comprising a membrane (2) having an application zone (5) for applying the liquid sample, at least one indicator zone which is able to interact with the blood group antigen, and at least one absorption region (3) which absorbs the liquid after passage through the indicator zone, wherein the indicator zone is positioned between the application zone (5) and an absorption region (3), **characterised in that** the indicator zone comprises an antibody directed against the blood group antigen, or a fragment thereof, and a binding element directed against the first antibody, wherein the first antibody is an incomplete antibody.

3. The device according to claim 1, wherein the device contains tubes filled with gel material.

4. The device according to any one of claims 1 to 3, wherein the blood group antigen is selected from the blood group antigens A, B, AB, D, C, E, c, e, Cw, K, k, Jka, Jkb, Fya, Fyb, M, N, S, s, P1, Kpa, Kpb, Lua, Lub, Lea, Leb, Mia, Dia, Jsa, Jsb, Coa, Cob, Wra and Xga.

5. The device according to claim 4, wherein the blood group antigen is selected from the blood group antigens k, S, Fya, Kpa, Kpb, Mia, Lua, Lub, Dia, Jsa, Jsb, Coa, Cob, Wra and Xga.

6. The device according to any one of claims 1 to 5, wherein the first antibody is an antibody of the IgG or IgA type, preferably of the IgG type.

7. The device according to any one of claims 1 to 6, wherein the binding element directed against the first antibody is selected from an antibody directed against the first antibody, or a fragment thereof, and a lectin or a fragment thereof.

8. The device according to claim 7, wherein the antibody is an anti-IgG or anti-IgA antibody, preferably a monoclonal anti-IgG antibody of the IgM class, or the lectin is protein A or protein G.

9. A device for simultaneously determining a first and a second blood group antigen in a liquid sample, comprising a membrane (2) having an application zone (5) for applying the liquid sample, at least two indicator zones which are able to interact with the blood group antigens, and at least one absorption region (3) which absorbs the liquid after passage through the indicator zone, wherein the indicator zones are positioned between the application zone (5) and the at least one absorption region (3), **characterised in that** (i) the first indicator zone comprises a first antibody directed against the first blood group antigen, or a fragment thereof, and a binding element directed against the first antibody, wherein the first antibody is an incomplete antibody,
and
(ii) the second indicator zone (a) comprises a first antibody directed against the second blood group antigen, wherein the first antibody is a complete antibody; or (b) comprises a first antibody directed against the second blood group antigen, wherein the first antibody is incomplete, and a binding element directed against said antibody.

10. The device according to claim 9, wherein the first blood group antigen is selected from the blood group antigens k, Fya, Kpa, Kpb, Lua, Lub, Mia, Dia, Jsa, Jsb, Coa, Cob, Wra, Xga, and S and the second blood group antigen is selected from A, B, AB, C, D, E, c, e, Cw, K, Lea, Leb, Jka, Jkb, Fyb, P1 and s.

11. The device according to any of claims 9 or 10, wherein the first antibody in the first indicator zone (i) and/or the first antibody in the second indicator zone (ii) is an antibody of the IgG or IgA type, preferably of the IgG type.

12. The device according to any one of claims 9 to 11, wherein the binding element directed against the first antibody in the first indicator zone (i) and/or in the second indicator zone (ii) is selected from an antibody directed against the first antibody, or a fragment thereof, and a lectin or a fragment thereof.

13. The device according to claim 12, wherein the binding element is an anti-IgG or anti-IgA antibody, or the lectin is protein A or protein G.

14. The device according to claim 13, wherein the binding element in the first indicator zone (i) and/or the second antibody in the second indicator zone (ii) is an IgM or IgG antibody.

15. The device according to any one of claims 9 to 14, wherein the first antibody directed against the first blood group antigen and the binding element directed against the first antibody in the first indicator zone (i) and/or the first antibody directed against the second blood group antigen and the second binding element directed against the first antibody in the second indicator zone (ii) are IgG antibodies.

16. The device according to any one of claims 9 to 15, wherein the second indicator zone (ii) comprises an IgM antibody directed against the second blood group antigen.

17. A method for producing a device according to any one of claims 1 to 16, comprising applying a first antibody directed against a blood group antigen, or a fragment thereof, and a binding element directed against the first antibody in the indicator zone, wherein the first antibody is an incomplete antibody.

18. The method for producing a device according to claim 17, wherein the first antibody and the binding element are applied separately from each other or as a mixture.

19. A method for determining at least one blood group antigen, comprising applying the sample to the application zone (5) of a membrane (2) of the device according to any one of the preceding claims 1 to 16, wherein said sample is present in an amount sufficient to cause the sample liquid to flow through the indicator zones towards the absorption region (3) and to cause the blood group antigens in the sample liquid to form a complex in the indicator zones.

20. The method according to claim 19, wherein the method does not comprise incubating the blood group antigen with an antibody before the blood group antigen is applied to the membrane.

21. The method according to claim 19 or 20, wherein the liquid sample consists of blood or blood components, preferably of whole blood, erythrocyte concentrate, coagulated blood or test liquid, such as control blood.

22. Use of the device according to any one of claims 1 to 8 or 9 to 16 for analysing blood, particularly for determining blood group antigens or antigen epitopes.

23. The use according to claim 22, wherein the blood group antigens or antigen epitopes are selected from A, B, AB, D, C, E, c, e, Cw, K, k, Jka, Jkb, Fya, Fyb, M, N, S, s, P1, Kpa, Kpb, Lua, Lub, Lea, Leb, Mia, Dia, Jsa, Jsb, Coa, Cob, Wra and/or Xga blood group antigens or antigen epitopes.

## Revendications

1. Dispositif destiné à déterminer un antigène de groupe sanguin dans un échantillon liquide, comprenant une matrice de séparation ayant au moins une zone indicatrice, **caractérisé en ce que** la zone indicatrice comprend un premier anticorps dirigé contre ledit antigène de groupe sanguin ou un fragment de celui-ci et un élément de liaison dirigé contre le premier anticorps, dans lequel le premier anticorps est un anticorps incomplet.

2. Dispositif selon la revendication 1, comprenant une membrane (2) ayant une zone de travail (5) pour l'application de l'échantillon liquide, au moins une zone indicatrice qui peut interagir avec l'antigène de groupe sanguin et au moins une zone d'absorption (3) qui absorbe le fluide après être passé dans la zone indicatrice, dans lequel la zone indicatrice se situe entre la zone de travail (5) et une zone d'absorption (3), **caractérisé en ce que** la zone indicatrice comprend un anticorps dirigé contre l'antigène de groupe sanguin ou un fragment de celui-ci et un élément de liaison dirigé contre le premier anticorps, dans lequel le premier anticorps est un anticorps incomplet.

3. Dispositif selon la revendication 1, dans lequel le dispositif contient des tubes remplis de matière en gel.

4. Dispositif selon l'une des revendications 1 à 3, dans lequel l'antigène de groupe sanguin est choisi parmi les antigènes de groupe sanguin A, B, AB, D, C, E, c, e, Cw, K, k, Jka, Jkb, Fya, Fyb, M, N, S, s, P1 , Kpa, Kpb, Lua, Lub, Lea, Leb, Mia, Dia, Jsa, Jsb, Coa, Cob, Wra et Xga.

5. Dispositif selon la revendication 4, dans lequel l'antigène de groupe sanguin est choisi parmi les antigènes de groupe sanguin k, S, Fya, Kpa, Kpb, Mia, Lua, Lub, Dia, Jsa, Jsb, Coa, Cob, Wra et Xga.

6. Dispositif selon l'une des revendications 1 à 5, dans lequel le premier anticorps est un anticorps du type IgG ou IgA, de préférence du type IgG.

7. Dispositif selon l'une des revendications 1 à 6, dans lequel l'élément de liaison dirigé contre le premier anticorps est choisi parmi un anticorps dirigé contre le premier anticorps ou un fragment de celui-ci, et une lectine ou un fragment de celle-ci.

8. Dispositif selon la revendication 7, dans lequel l'anticorps est un anticorps anti-IgG ou anti-IgA, de préférence un anticorps anti-IgG monoclonal de la classe IgM, ou la lectine protéine A ou protéine G.

9. Dispositif pour déterminer simultanément un premier et un second antigènes de groupe sanguin dans un échantillon liquide, comprenant une membrane (2) ayant une zone de travail (5) pour l'application de l'échantillon liquide, au moins deux zones indicatrices qui peuvent interagir avec ledit antigène de groupe sanguin et au moins une zone d'absorption (3) qui absorbe le fluide après être passé dans la zone indicatrice, dans lequel les zones indicatrices se situent entre la zone de travail (5) et la au moins une zone d'absorption (3), **caractérisé en ce que** (i) la première zone indicatrice comprend un premier anticorps dirigé contre le premier antigène de groupe sanguin ou un fragment de celui-ci et un élément de liaison dirigé contre le premier anticorps, dans lequel le premier anticorps est un anticorps incomplet et
(ii) la seconde zone indicatrice (a) comprend un premier anticorps dirigé contre le second antigène de groupe sanguin, dans lequel le premier anticorps est un anticorps complet ; ou (b) comprend un premier anticorps dirigé contre le second antigène de groupe sanguin, dans lequel le premier anticorps est incomplet, et un élément de liaison dirigé contre cet anticorps.

10. Dispositif selon la revendication 9, dans lequel le premier antigène de groupe sanguin est choisi parmi les antigènes de groupe sanguin k, Fya, Kpa, Kpb, Lua, Lub, Mia, Dia, Jsa, Jsb, Coa, Cob, Wra, Xga et S, et le second antigène de groupe sanguin est choisi parmi A, B, AB, C, D, E, c, e, Cw, K, Lea, Leb, Jka, Jkb, Fyb, P1 et s.

11. Dispositif selon l'une des revendications 9 ou 10, dans lequel le premier anticorps dans la première zone indicatrice (i) et/ou le premier anticorps dans la seconde zone indicatrice (ii) est un anticorps du type IgG ou IgA, de préférence du type IgG.

12. Dispositif selon l'une des revendications 9 à 11, dans lequel l'élément de liaison dirigé contre le premier anticorps dans la première zone indicatrice (i) et/ou dans la seconde zone indicatrice (ii) est choisi parmi un anticorps dirigé contre le premier anticorps ou un fragment de celui-ci, et une lectine ou un fragment de celle-ci.

13. Dispositif selon la revendication 12, dans lequel l'élément de liaison est un anticorps anti-IgG ou anti-IgA ou la lectine protéine A ou protéine G.

14. Dispositif selon la revendication 13, dans lequel l'élément de liaison dans la première zone indicatrice (i) et/ou le second anticorps dans la seconde zone indicatrice (ii) est un anticorps IgM ou IgG.

15. Dispositif selon l'une des revendications 9 à 14, dans lequel le premier anticorps dirigé contre le premier antigène de groupe sanguin et l'élément de liaison dirigé contre le premier anticorps dans la première zone indicatrice (i) et/ou le premier anticorps dirigé contre le second antigène de groupe sanguin et le second élément de liaison dirigé contre le premier anticorps dans la seconde zone indicatrice (ii) sont des anticorps IgG.

16. Dispositif selon l'une des revendications 9 à 15, dans lequel la seconde zone indicatrice (ii) comprend un anticorps IgM dirigé contre le second antigène de groupe sanguin.

17. Procédé de fabrication d'un dispositif selon l'une des revendications 1 à 16, comprenant l'application d'un premier anticorps dirigé contre un antigène de groupe sanguin ou d'un fragment de celui-ci et d'un élément de liaison dirigé contre le premier anticorps dans la zone indicatrice, dans lequel le premier anticorps est un anticorps incomplet.

18. Procédé de fabrication d'un dispositif selon la revendication 17, dans lequel le premier anticorps et l'élément de liaison sont appliqués séparément l'un de l'autre ou sous forme de mélange.

19. Procédé pour déterminer au moins un antigène de groupe sanguin, comprenant l'application de l'échantillon sur la zone de travail (5) d'une membrane (2) du dispositif selon l'une des revendications 1 à 16 précédentes, dans lequel cet échantillon est présent en quantité suffisante pour amener le liquide d'échantillon à s'écouler en direction de la zone d'absorption (3) à travers les zones indicatrices, et amener des antigènes de groupe sanguin dans le liquide d'échantillon à former un complexe dans les zones indicatrices.

20. Procédé selon la revendication 19 , dans lequel le procédé ne comprend pas l'incubation de l'antigène de groupe sanguin avec un anticorps avant l'application de l'antigène de groupe sanguin sur la membrane.

21. Procédé selon la revendication 19 ou 20, dans lequel l'échantillon liquide est constitué de sang ou de constituants du sang, de préférence de sang total, de concentré d'érythrocytes, de sang coagulé ou d'un liquide de test, tel que du sang témoin.

22. Utilisation du dispositif selon l'une des revendications 1 à 8 ou 9 à 16 pour l'analyse de sang, en particulier pour la détermination d'antigènes de groupe sanguin ou d'épitopes d'antigène.

23. Utilisation du dispositif selon la revendication 22, dans laquelle les antigènes de groupe sanguin ou les épitopes d'antigène sont choisis parmi les antigènes de groupe sanguin ou des épitopes d'antigène A, B, AB, D, C, E, c, e, Cw, K, k, Jka, Jkb, Fya, Fyb, M, N, S, s, P1 , Kpa, Kpb, Lua, Lub, Lea, Leb, Mia, Dia, Jsa, Jsb, Coa, Cob, Wra et/ou Xga.
